# EUROPEAN PATENT SPECIFICATION

(11) **EP 1 268 441 B1**
(45) Date of publication and mention of the grant of the patent: **09.05.2007**
(21) Application number: 01921685.2
(22) Date of filing: 27.03.2001
(51) Int. Cl.: C07D 233/54, A61K 31/4164, A61P 43/00, C07D 209/88

(54) **AN INTERMEDIATE AND PROCESS FOR ITS SYNTHESIS**
EINE ZWISCHENVERBINDUNG UND VERFAHREN ZU DEREN HERSTELLUNG
INTERMEDIAIRE ET PROCEDE DE SYNTHESE CORRESPONDANT

(30) Priority: 28.03.2000 HU 0001287
(43) Date of publication of application: 02.01.2003
(73) Proprietor: RICHTER GEDEON VEGYÉSZETI GYÁR RT, 1103 Budapest X (HU)
(72) Inventor: CZIBULA, Lászlo, H-1103 Budapest (HU); DOBAY, Lászlo, H-1073 Budapest (HU); GREINER, István, H-1221 Budapest (HU); WERKNE PAPP, Eva, H-1103 Budapest (HU); SZANTAY, Csaba, H-1113 Budapest (HU); GAZDAG, Mária, H-1112 Budapest (HU); TARKANYI, Gábor, H-2040 Budaors (HU); ZSOLDOSNE BABJAK, Monika, H-1106 Budapest (HU); MIHALYFI, Katalin, H-1084 Budapest (HU)
(74) Representative: Bublak, Wolfgang
(86) International application number: PCT/HU2001/000035
(87) International publication number: WO 2001/072716

(56) References cited:
- EP-A- 0 595 111

## Description

The invention relates to a new intermediate, the (2-methyl-1-imidazolyl)-9-methyl-3-hydroxymethyl-1,2,3,9-tetrahydro-4H-carbazol-4-on-3-glyoxylate of formula (I).

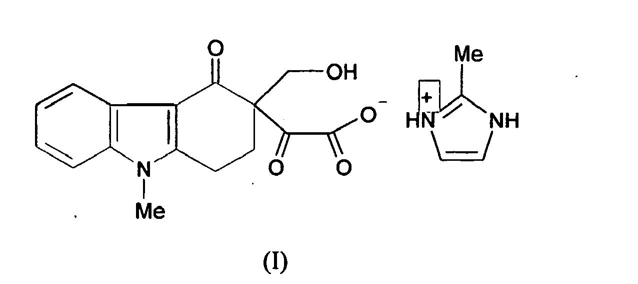

The invention also relates to the process for the synthesis of the above compound.

It is well known that ondansetron (chemical name 1,2,3,9-tetrahydro-9-methyl-3-[(2-methyl-1H-imidazol-1-yl)-methyl]-4H-carbazolon-4-on), together with its physiologically acceptable salts and solvates, may be used in the treatment of a variety of conditions, including the nausea and vomiting induced by cancer chemotherapy and radiotherapy (as described in EP Pat. 226,266). Several chemical processes are known from the literature for the synthesis of ondansetron; see for example GB Pat. 2,153,821 or 2,192,885 or EP Pat. 595,111.

It was found, as a basis of our invention, that besides the intermediates described, a new, useful intermediate can be synthesised during the investigation of the synthetic pathway published in the European patent number 595,111, which summarises the results of our own research. This new intermediate can simply be converted into the known drug substance, ondansetron hydrochloride dihydrate.

According to the above mentioned facts the invention relates to (2-methyl-1-imidazolyl)-9-methyl-3-hydroxymethyl-1,2,3,9-tetrahydro-4H-carbazol-4-on-3-glyoxylate of formula (I).

The invention also relates to the process for the synthesis of (2-methyl-1-imidazolyl)-9-methyl-3-hydroxymethyl-1,2,3,9-tetrahydro-4H-carbazol-4-on-3-glyoxylate of formula (I).

According to our invention a known compound (see: EP Pat. 595,111); 9-methyl-3-hydroxymethyl-1,2,3,9-tetrahydro-4H-carbazol-4-one-3-glyoxilic acid lactone of formula (II) 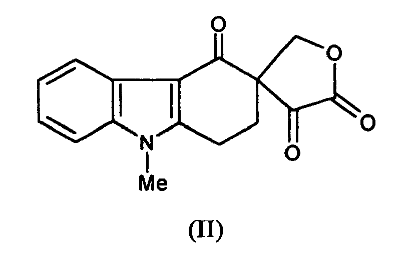 is reacted with 2-methylimidazol and the product [formula (I)] is isolated.
The invention is illustrated by the following examples.

### Example 1.

Synthesis of (2-methyl-1-imidazolyl)-9-methyl-3-hydroxymethyl-1,2,3,9-tetrahydro-4H-carbazol-4-on-3-glyoxylate (I)
The reaction pathway:

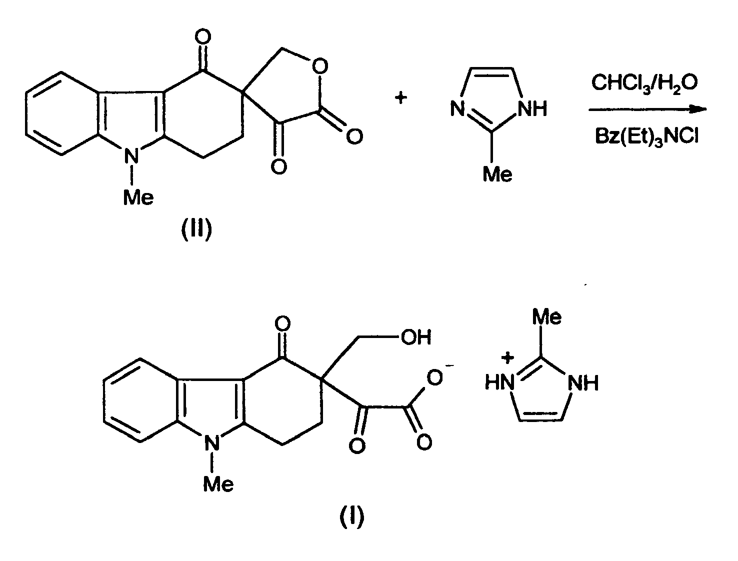

### Detailed description:

To a suspension of 22.0 g (0.077 mol) of 9-methyl-3-hydroxymethyl-1,2,3,9-tetrahydro-4H-carbazol-4-on-3-glyoxylic acid lactone and 7.2 g (0.088 mol) of 2-methyl-imidazol in 240 ml of chloroform and 120 ml of water, 1.2 g of benzyltriethylammonium chloride was added and the mixture was stirred at 22-24°C for 3 hours. The precipitated crystals were filtered off, washed three times with 50 ml of water and dried at 60°C until the weight has stabilised to yield 28 g (94%) of the title compound. Mp.: 134-136°C.

### Example 2

Synthesis of 2-methyl-1-imidazolyl-9-methyl-3-hydroxymethyl-1,2,3,9-tetrahydro-4H-carbazol-4-on-3-glyoxylate (I) The reaction pathway:

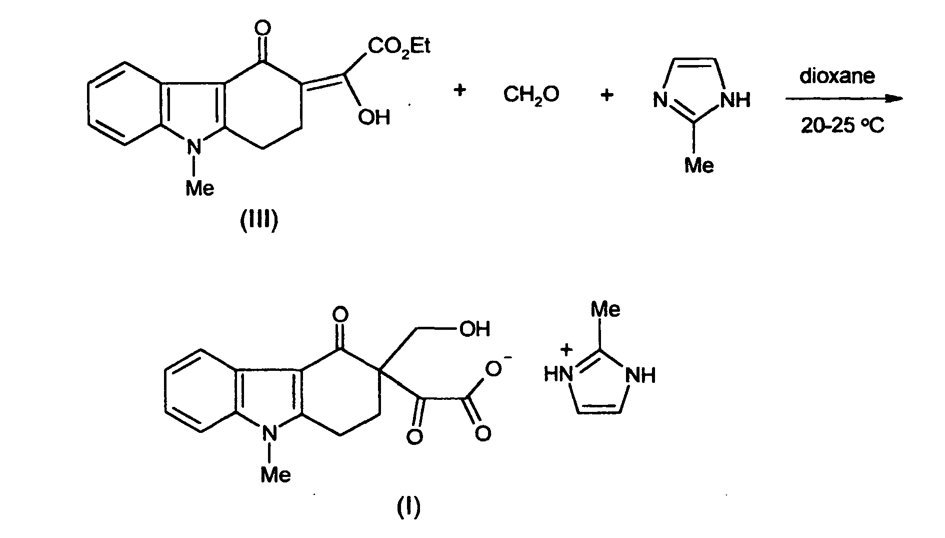

### Detailed description:

To a solution of 15 g (0.05 mol) of 9-methyl-3-ethooalyl-1,2,3,9-tetrahidro-4H-carbazol-4-on (III) in 150 ml of dioxane 5.5 g of 35% formaldehyde solution and 12 g (0.15 mol) of 2-methylimidazol were added and the mixture was stirred at 20-25°C for 3-4 h. The reaction mixture was then allowed to cool to 5°C, the crystals were filtered off, washed twice with 25 ml of dioxane and dried below 60°C to yield 18 g (94%) of the title compound. Mp.: 132-135°C.

### Example 3

### Synthesis of ondansetron hydrochloride dihydrate

To a suspension 10 g (0,026 mol) of (2-methyl-1-imidazolyl)-9-methyl-3-hydroxymethyl-1,2,3,9-tetrahydro-4H-carbazol-4-on-3-glyoxylate in 150 ml of dioxane 4,3 g (0,052 mol) of 2-methyl-imidazol was added. After stirring at 100 °C in nitrogen atmosphere for 1 hour, the reaction mixture was cooled to 25-30 °C, 45 ml of water was added and stirred at 20 °C for 1 hour. The crystals were filtered off, washed twice with 30 ml of water, to give 5,3 g of ondansetron base. To the crude base was added 42 ml of isopropanole, 5 ml of water and 1,6 ml of concentrated hydrochloric acid, dissolved at 80 °C, then cooled to 5 °C, stirred at the same temperature for 2 hours, the separated crystals were filtered off, washed twice with 15 ml of a mixture isopropanole-water 95:5, and dried at room temperature to yield 5,9 (65 %) of the title compound. Water content is: 9,5-10 %.

### NMR, MS and IR spectroscopic characterization

| **NMR** | |
|---|---|
| Name of the compound: | (2-methyl-1-imidazolyl)-9-methyl-3-hydroxymethyl-1,2,3,9-tetrahydro-4H-carbazol-4-on-3-glyoxylate |
| Instrument: | Varian *INOVA-300* NMR spectrometer (¹H: 300 MHz) |
| Solvent: | DMSO-d₆ |
| Temperature: | 30°C |
| Reference: | δ_{TMS} = 0.00 ppm |
| Spectrum number: | H-13853; C-6848 |

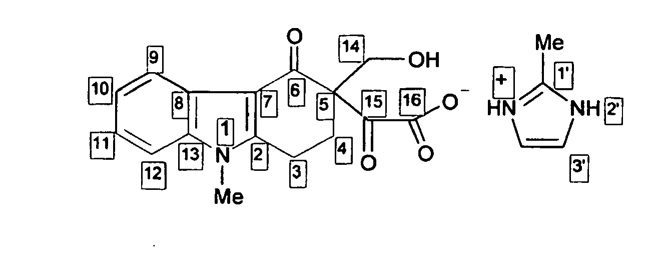

*¹H NMR* 2.24-2.36 m (1H) [H_{y}-4]; 2.47 s (3H) [1'-Me]; 2.67-2.76 m (1H) [Hₓ-4]; 3.06-3.14 m (2H) [H₂-3]; 3.70 s (3H) [1-Me]; 3.87 d (J = 10.8 Hz) (1H) [H_{y}-14]; 3.94 d (J = 10.8 Hz) (1H) [Hₓ-14]; 7.15-7.26 m (2H) [H-10, H-11]; 7.32 s (2H) [3'=CH]; 7.50 dm (1H) [H-12]; 7.98 dm (1H) [H-9]; 7.00-8.80 vbr (ca. 4H) [chemically exchangeable protons: 2'-NH, 14-OH, H₂O].
*¹³C NMR* 11.4 (1'-Me); 19.2 (C-3); 27.5 (C-4); 29.6 (1-Me); 62.7 (C-5); 63.8 (C-14); 110.0 (C-12); 111.4 (C-7); 119.0 (C-3'); 120.2 (C-9); 121.8 (C-10); 122.4 (C-11); 124.5 (C-8); 137.3 (C-13); 143.8 (C-1'); 152.0 (C-2); 168.4 (C-16); 189.5 (C-6); 202.9 (C-15).

| **MS** | |
|---|---|
| Name of the compound: | (2-methyl-1-imidazolyl)-9-methyl-3-hydroxymethyl-1,2,3,9-tetrahydro-4H-carbazol-4-on-3-glyoxylate |
| Instrument: | Finnigan MAT 95SQ |
| Method of ionization: | FAB |
| FAB gun: | Cs⁺, 20kV |
| FAB matrix: | 3-nitrobenzyl alcohol |
| Spectrum number: | FM-5262/+FAB FM-5263/-FAB FM-5264/ daughter ion experiment |

+FAB experiment:
m/z (rel. int.%): 384 (8.4, MH+); 302 (4.8); 229 (15); 83 (100)

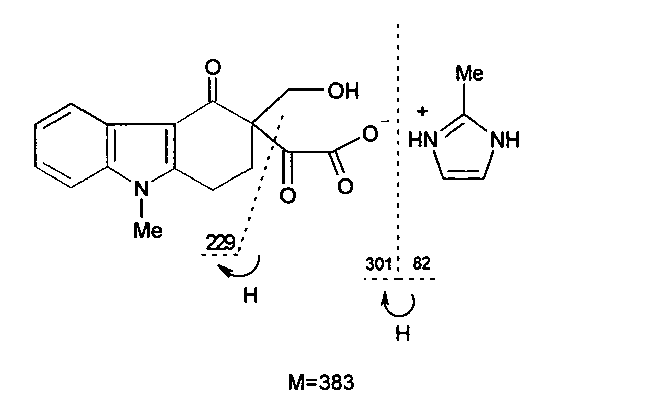

| **m/z** | **Ions]⁺** |
|---|---|
| 302 | MH-C₄H₆N₂ |
| 229 | MH-C₄H₇N₂-C₂O₃ |
| 83 | C₄H₇N₂ |

The +FAB spectrum verifies that the molecule contains a cation (C⁺) and an anion (A⁻).

### FAB daughter ion experiment:

According to the FAB daughter ion experiment the ions m/z 83 and 302 observed in the +FAB spectrum are the primary fragments of the protonated molecular ion (m/z 384 [C⁺A⁻ + H⁺]⁺.

### -FAB experiment:

In the -FAB spectrum the anion (m/z 300, A⁻) and the ion [2A⁻+H⁺] formed during the experiment can be observed.

| **IR** | |
|---|---|
| Name of the compound: | (2-methyl-1-imidazolyl)-9-methyl-3-hydroxymethyl-1,2,3,9-terahydro-4H-carbazol-4-on-3-glyoxylate |
| Instrument: | PERKIN-ELMER 1000 spectrophotometer |
| Phase: | KBr pellet |
| Resolution: | 4 cm⁻¹ |
| Spectrum number: | 7253 |

The most characteristic IR bands (cm⁻¹):

| | |
|---|---|
| O-H | 3306 |
| N⁺-H | 3200-2100 |
| C=O | 1691,1626 |
| COO⁻ | 1598 |
| Ar | 754 |

Other significant IR bands (cm⁻¹): 3115, 2713, 1480, 1455, 1318, 1065, 1029, 915, 514.

## Claims

1. (2-methyl-1-imidazolyl)-9-methyl-3-hydroxymethyl-1,2,3,9-tetrahydro-4H-carbazol-4-on-3-glyoxylate of formula (I).

2. Process for the preparing of (2-methyl-1-imidazolyl)-9-methyl-3-hydroxymethyl-1,2,3,9-tetrahydro-4H-carbazol-4-on-3-glyoxylate of formula (I), **characterized by** 9-methyl-3-hydroxymethyl-1,2,3,9-tetrahydro-4H-carbazol-4-on-3-glyoxylic acid lactone is reacted with 2-methylimidazol, followed by isolation of the product of formula (I).

3. Use of (2-methyl-1-imidazolyl)-9-methyl-3-hydroxymethyl-1,2,3,9-tetrahydro-4H-carbazol-4-on-3-glyoxylate of formula (I) for the production of ondansetron hydrochloride dihydrate.

## Patentansprüche

1. (2-Methyl-1-imidazolyl)-9-methyl-3-hydroxymethyl-1,2,3,9-tetrahydro-4H-carbazol-4-on-3-glyoxylat der Formel (I).

2. Verfahren zur Herstellung von (2-Methyl-1-imidazolyl)-9-methyl-3-hydroxymethyl-1,2,3,9-tetrahydro-4H-carbazol-4-on-3-glyoxylat der Formel (I), **dadurch gekennzeichnet, dass** 9-Methyl-3-hydroxymethyl-1,2,3,9-tetrahydro-4H-carbazol-4-on-3-glyoxylsäurelacton mit 2-Methylimidazol umgesetzt wird, gefolgt von einer Isolierung des Produkts der Formel (I).

3. Verwendung von (2-Methyl-1-imidazolyl)-9-methyl-3-hydroxymethyl-1,2,3,9-tetrahydro-4H-carbazol-4-on-3-glyoxylat der Formel (I) zur Herstellung von Ondansetron-hydrochlorid-dihydrat.

## Revendications

1. (2-Méthyl-1-imidazolyl)-9-rnéthyl-3-hydroxyméthyl-1,2,3,9-tétrahydro-4H-carbazol-4-one-3-glyoxylate de formule (I).

2. Procédé de préparation de (2-méthyl-1-imidazolyl)-9-méthyl-3-hydroxyméthyl-1,2,3,9-tétrahydro-4H-carbazol-4-one-3-glyoxylate de formule (I), **caractérisé par** la mise en réaction de la lactone de l'acide 9-méthyl-3-hydroxyméthyl-1,2,3,9-tétrahydro-4H-carbazol-4-one-3-glyoxylique avec le 2-méthylimidazole, suivie de l'isolement du produit de formule (I).

3. Utilisation du (2-méthyl-1-imidazolyl)-9-méthyl-3-hydroxyméthyl-1,2,3,9-tétrahydro-4H-carbazol-4-one-3-glyoxylate de formule (I) pour la production de chlorhydrate d'ondansétron dihydrate.
